# EUROPEAN PATENT SPECIFICATION

(11) **EP 1 995 329 B1**
(45) Date of publication and mention of the grant of the patent: **25.09.2013**
(21) Application number: 08008537.6
(22) Date of filing: 06.05.2008
(51) Int. Cl.: C12Q 1/68

(54) **Method for sequencing nucleic acids**
Verfahren zur Sequenzierung von Nukleinsäuren
Procédé de séquençage d'acides nucléiques

(30) Priority: 10.05.2007 JP 2007125273; 27.06.2007 JP 2007168866
(43) Date of publication of application: 26.11.2008
(73) Proprietor: Sony Corporation, Tokyo 108-0075 (JP)
(72) Inventor: Kishii, Noriyuki, Tokyo (JP); Ichimura, Mari, Tokyo (JP); Abe, Tomoteru, Tokyo (JP); Nakagawa, Kazuhiro, Tokyo (JP); Yasuda, Akio, 10785 Berlin (DE); Shiono, Mayumi, Tokyo (JP); Ohnishi, Michihiro, Tokyo (JP); Yamamoto, Takuro, Tokyo (JP); Takeda, Minoru, Tokyo (JP)
(74) Representative: Müller - Hoffmann & Partner

(56) References cited:
- EP-A2- 1 018 365
- WO-A1-01/62699
- WO-A1-99/60170
- WO-A2-01/14589
- WO-A2-03/060460
- WO-A2-2005/074569
- US-A1- 2003 162 181
- US-A1- 2004 175 842
- US-A1- 2006 105 352

## Description

### CROSS REFERENCES TO RELATED APPLICATIONS

The present invention contains subject matter related to Japanese Patent Application JP 2007-168866 filed in the Japan Patent Office on June 27, 2007.

### BACKGROUND OF THE INVENTION

### 1. Field of the Invention

This invention relates to a novel technology useful especially in biochemical analysis technologies. More specifically, the present invention concernes a method for determining a base sequence by using a bead set which permits identification of individual beads.

### 2. Description of the Related Art

In the field of biochemical analysis technologies and the like, particulate carriers generally called "beads (microbeads)" may be used in certain instances. As a most extensively employed biochemical application of beads, illustrative can be the use of silica beads or polymer beads as packing materials in separation columns for liquid chromatography.

By coupling antibodies, avidins or the like to the surfaces of beads, they are also used for the trapping, separation, purification or the like of target substances from biological samples. For example, the method that beads, on which an antibody to a specific amino acid sequence in a protein has been immobilized beforehand is mixed in an extract of cells to trap the target protein (or its complex) has become a useful method for the analysis of interaction of the protein (ISOBE, Toshiaki and TAKAHASHI, Nobuhiro: "Extra Issue - Experimental Lecture 2 for Postgenome Age, Proteome Analysis Methods", p. 166-174, 2000, Yodosha Co., Ltd.).

Also known are methods that use "magnetic beads". The use of magnetic beads, on which an antibody to a microbial antigen has been immobilized, for the trap and detection of a target microorganism from a sample through an antigen-antibody reaction can be mentioned as an example (Japanese Patent Laid-open Nos. 2006-017554, and 2001-004631, etc.). A still further method has also been proposed to combine magnetic beads with a microchannel system to conduct separation of cells (Japanese Patent Laid-open No. 2006-006166).

A yet still further method has also been proposed. According to this method, an interaction between substances (e.g., hybridization) is allowed to proceed on the surfaces of beads. An electric field is then applied to the place of the interaction such that the beads are caused to migrate in accordance with charge amounts (coulomb forces) of the respective substances held on the beads for the analysis of the existence or non-existence of the interaction between the substances (Japanese Patent Laid-open No. 2003-302373).

In addition, an analysis technology has already found practical utility, which makes use of a set of beads (which is composed of as many as 100 types of beads at the maximum) in which these different types of beads can be distinguished from one type to another by differentiating the color of light, which are to be emitted from the types of beads, depending on combinations of two fluorescent dyes at varied ratios ("LUMINEX" systems (trademark); visit http://hitachisoft.jp/dnasis/luminex/microbead/beads.html ). This method, however, needs an accurate detection of an emission because the different types of beads are distinguished depending on differences in emission intensity.

WO 99/60170 discloses a plurality of array beads wherein each bead has immobilized thereon a compound that is potentially capable of binding to a target analyte. In addition, so-called marker beads are provided located between the beads.

From US 2003/0162181 A1 a mixture of optically distinguishable materials conjugated with DNA sequence recognition units is known. This unit is hybridized to target DNA. The optically distinguishable material comprises microparticles having different shapes.

WO 2005/074569 A2 teaches shape encoded particles with specific distinct shapes. whereas these shapes are used as particle identifiers.

US 2004/0175842 A1 discloses microbeads wherein the microbead surface can be suitably modified for molecular recognition and an encoding method is provided that identifies uniquely the class corresponding to a particular microbead.

US 2006/0105352 A1 discloses a target analyte which is provided in a suspending solution containing polymeric particles marked with a probe wherein said probe has an affinity for said target analyte. Said polymeric particles have a distinct shape, for example a curvilinear shape, a spherical shape, a donut shape, an elliptical shape, a rectangular shape, a cubic shape or a rod shape.

In WO 03/060460 A2, target libraries are immobilized on encoded units wherein one possibility for encoding would be the use of units of different size and/or shape, colour or fluorescence or by molecular tags.

WO 01/14589 A2 discloses discrete solid-phase supports, i.e. microparticles. Nucleic acid probes are bound thereto, wherein each distinct type of a probe is bound to a fluorescently addressable set of microparticles. A plurality of fluorescently addressable microparticles are provided.

WO 01/62699 concerns optically encoded particles which are coated with a reactive chemistry or linking chemistry which is used to attach a probe molecule. Each particle has a unique set of characters which can be identified.

EP 1 018 365 A2 discloses a combinatorial chemistry solid support particle marked with a machine readable code, wherein the support particle comprises a first phase and a second phase containing the machine readable code. The second phase is substantially encapsulated with the first phase. The machine readable code recites in the shape of the second phase.

### SUMMARY OF THE INVENTION

As has been described above, the development of application technologies for beads in the field of biochemistry is still in progress at present, and its applications are expected to expand further in the future.

On the other hand, there is an outstanding demand for novel technologies which can contribute to speed-up, the realization of comprehensive analyses, efficiency improvements, accuracy improvements and the like in the field of various biochemical analysis technologies. Primary technical demands include, for example, speed-up of the reading time in DNA sequencing technologies and work efficiency improvements in drug screening technologies. In recent years, it has been reported that ribonucleic acids (RNAs), which do not encode proteins, exist and control the functions of proteins, and it has also been suggested that certain DNA fragments act as a cause in the onset of rheumatism. There is, accordingly, an outstanding demand for technologies useful in the comprehensive and highly-accurate detection of the sequences of DNAs or RNAs which exist as fragments in cells.

It is desirable to provide novel biotechnical analysis technologies which make it possible to meet the above-described technical needs. More specifically, primary needs of the present invention are to provide a bead identification technology capable of dramatically increasing the number of types of beads distinguishable from one type to another and also applied analysis technologies making use of the bead identification technology.

This is achieved by the subject matter of claim 1.

According to the present invention, a bead set composed of plural subsets of beads is used, wherein the beads in each subset are each provided with a surface permitting immobilization thereon of a substance that takes part in a predetermined corresponding reaction or interaction, and the individual beads in the bead set are provided with different cores, respectively, such that the individual beads in the bead set can be distinguished into the plural subsets through an analysis of captured images of the individual beads in the bead set by a computer. As the physical elements, the profiles of the beads, the profiles of the core portions of the beads, identifiers and the like can be exemplified.
It is to be noted that the term "reaction or interaction" as used herein widely means chemical bonding, including non-covalent bonding, covalent bonding and hydrogen bonding between substances, and dissociation, and broadly includes, for example, specific bonding or association such as hybridization as complementary bonding between nucleic acids (nucleotide chains) and macromolecule-macromolecule, macromolecule-micromolecule and micromolecule-micromolecule bonding or association.

The individual beads in the bead set are contrived to comprise core portions and shell portions as outer layers for the core portions, respectively, and the core portions may be provided with shapes different from one subset to another, respectively, such that the individual beads in the bed set can be distinguished into the plural subsets through an analysis of the shapes of the core portions from information on captured images of the individual beads in the bead set by a computer.

According to the present invention, there is provided a method for determining a base sequence of a target nucleic acid strand, which comprises using at least the above-described bead set.

The bead set used according to the present invention may generally be composed of a large number of subsets of beads which can be distinguished from one subset to another, and therefore, can be used as a useful tool capable of achieving speed-up, the realization of comprehensive analyses, efficiency improvements, accuracy improvements and the like in various analysis technologies.

### BRIEF DESCRIPTION OF THE DRAWINGS

FIG. 1 is a diagram showing a bead set representing background art;
FIG. 2 is a diagram illustrating the bead set according to the present invention;
FIG. 3 is a diagram depicting a bead set representing background art;
FIG. 4 is a diagram showing an illustrative process flow for explaining one example of a production process for the bead set of FIG. 1;
FIG. 5 is a plan view showing one example of a photomask useful in the production process;
FIGS. 6A to 6C are plan views illustrating examples of light-transmitting areas in the photomask, in which light-shielding portions are arranged further;
FIG. 7 is a conceptual diagram illustrating that a polymerase extension reaction using a primer proceeded only on specific beads;
FIG. 8 is a schematic block diagram illustrating one example of a system configuration capable of performing a measurement of bead shapes and a measurement of fluorescence intensities;
FIG. 9 is a flowchart relating to identification and assessment of a bead shape;
FIG. 10 is a conceptual diagram of a procedure relating to the identification and assessment of the bead shape;
FIG. 11 is a diagram histogrammatically presenting fluorescence intensities measured corresponding to totally seven types of beads, the types being different in the shape of each core portion, respectively;
FIG. 12 is a schematic diagram illustrating a concept for determining the sequence of a target DNA oligomer by using base sequences immobilized on a bead set according to the embodiment of the present invention; and
FIG. 13 is a schematic diagram as background art illustrating a concept for detecting hybridization by using an illustrative bead set.

### DETAILED DESCRIPTION OF THE PREFERRED EMBODIMENTS

With reference to the accompanying drawings, a description will hereinafter be made about certain preferred embodiments of the present invention.

### (1) Beads

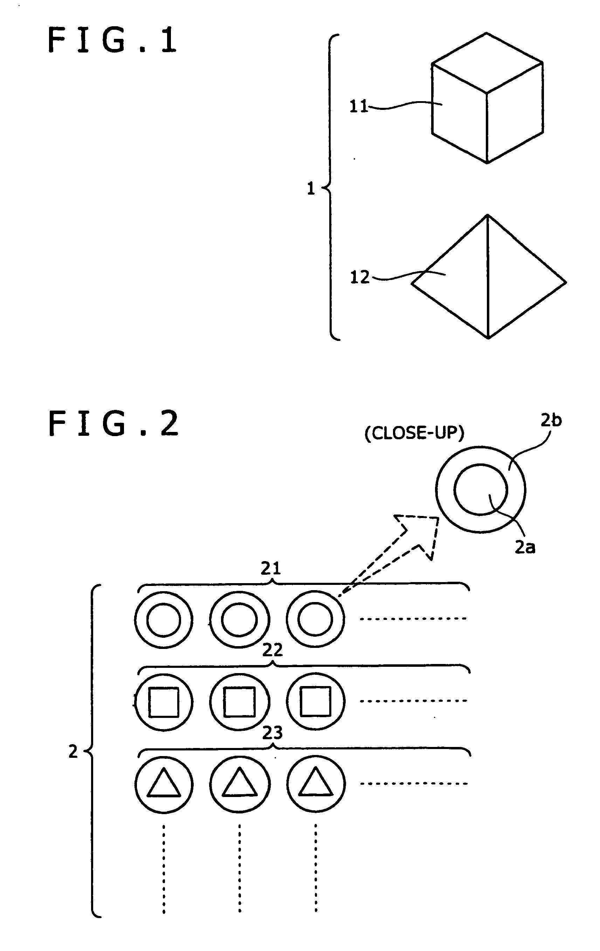

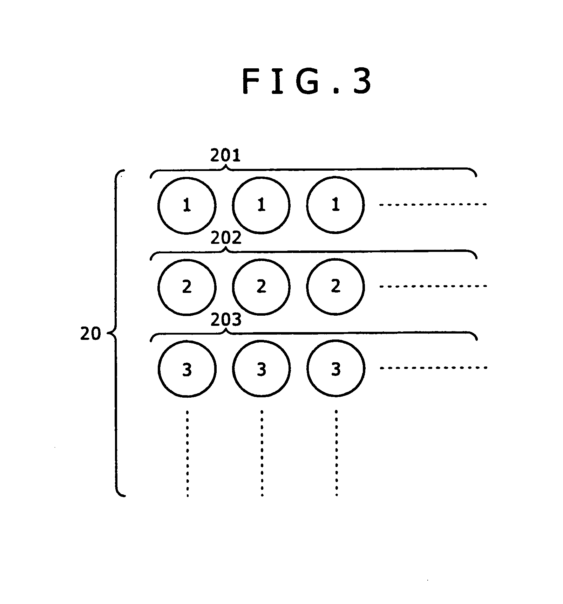

FIG. 1 is a diagram showing a bead set representing background art, specifically "a bead set composed of plural subsets of beads, wherein the beads in each subset are each provided with a surface permitting immobilization thereon of a substance that takes part in a predetermined corresponding reaction or interaction, and the individual beads in the beat set are provided with different physical elements, respectively, such that the individual beads in the bead can be distinguished into the plural subsets through an analysis of captured images of the individual beads in the bead set by a computer". FIG. 2 is a diagram illustrating the bead set according to the present invention, and FIG. 3 is a diagram depicting an other bead set representing background art.

A description will be now made from a bead set 1 shown in FIG. 1. This bead set 1 is composed of plural types of beads, which are different in stereoscopic shape such that they can be distinguished from one type to another based on image processing by a computer. For example, the bead set 1 is composed of cubic (regular hexahedral) beads 11 and triangular pyramidal (regular tetrahedral) beads 12 as collectively indicated by numeral 1 in FIG. 1. It is to be noted that, although these two types of beads are illustrated for easier understanding of this embodiment, no particular limitation is imposed on the number of types of beads is not particularly limited and can be suitably set depending on the purpose and need.

As a modification of the bead set 1, a bead set may be composed of plural types of beads having different two-dimensional shapes (e.g., a circle, a square, a triangle, etc.) such that the plural types of beads can be distinguished from one type to another based on image processing by a computer (not specifically shown as a figure).

It may also be contrived to apply predetermined identifiers to the bead set 1 or the modification. These identifiers are applied to permit distinction of the types of beads by subjecting their images, which are to be captured by a CCD camera or the like, to image processing by a computer.

On the specific kinds of the identifiers, on the other hand, no particular limitation is imposed. These identifiers can be suitably selected and adopted from numbers, number strings, characters, character strings, graphics, patterns, bar codes, additional shape portions, and combinations of two or more of these identifiers. As to areas to which the identifiers are applied, no particular limitations are imposed insofar as the identifiers can be distinguished from images captured by a CCD camera or the like. For example, these areas can be the surfaces of the beads or can be inner portions of the beads.

The beads which make up the bead set 1 are all contrived such that their surfaces each permits immobilization of a substance which takes part in a predetermined corresponding reaction or interaction. Coating treatment or chemical treatment may be applied to the surfaces of the beads 11,12 as needed to permit immobilization or desired substances, respectively. For example, the beads may be treated such that desired substances can be immobilized on the surfaces of the beads, respectively, through chemical bonds such as such as disulfide bonds, amide bonds, avidin-biotin bonds or the like.

Next, beads in a bead set 2 illustrated in FIG. 2 are commonly provided with a core portion 2a, which makes up a core structure portion of each bead, and a shell portion 2b, which is formed to enclose the core portion 2a at the circumference thereof, as appreciated from a bead depicted as a close-up in FIG. 2 (see the sketch shown forward of a dotted arrow in FIG. 2).

The core portions 2a are formed in various shapes for the purpose that they make it possible to classify the individual beads of the bead set 2 into plural subsets. For example, the core portions 2a are formed, as shapes as viewed in front view, in polygons, e.g., a triangle, quadrilaterals such as a square and a rectangle, pentagon and hexagon, and starbursts, in addition to a circle. Taking a circle as an example, the size of each core portion 2a may be assumed to be, for example, 50 µm or so in diameter.

As the shape of each core portion 2a, a two-dimensional shape, three-dimensional shape or the like can be chosen as desired insofar as it permits distinction of an image of the core portion. Further, the size of each core portion 2a can also be determined as desired depending on the purpose or application.

The shell portion 2b serves as a surface layer portion for each bead, and is a portion formed by applying a synthetic resin material (e.g., polystyrene) to the corresponding core portion 2a by coating treatment which will be described subsequently herein. No particular limitation is imposed on the profile of the shell portion 2b, and as an example, it can be a spherical shape or a shape closely resembling the spherical shape. More desirably, the individual beads which make up the bead set 2 can have the same surface area.

FIG. 2 shows, by way of example, a bead subset 21 in which the core portions 2a have a circular shape as viewed in front view, a bead subset 22 in which the core portions 2a have a square shape as viewed in front view, and a bead subset 23 in which the core portions 2a have a triangular shape as viewed in front view. The individual beads of the bead set 2 can each be distinguished into one of the bead subset 21, bead subset 22 and bead subset 23 by processing an image of the bead, which is to be captured by a CCD camera or the like, and analyzing its information, both, by a computer. It is to be noted that one or more additional bead subsets (not shown) different in the shape of each core portion 2a may also be provided as desired depending on the purpose.

Referring next to FIG. 3, a description will be made about a bead set 20. As shown in FIG. 3, this bead set 20 is characterized in that predetermined identifiers have been applied. These identifiers were applied to permit distinction of its beads from one subset to another by subjecting their images, which are to be captured by a CCD camera or the like, to information processing by a computer.

As to the specific kinds of the identifiers, they are not limited to such single numbers as shown in FIG.3, and number strings, characters, character strings, graphics, patterns, bar codes, additional shape portions, or combinations of two or more of these identifiers can be suitably adopted. It is to be noted that in FIG. 3, a bead subset 201 in which an identifier (number) 1 is applied, a bead subset 202 in which another identifier (number) 2 is applied and a bead subset 203 in which a further identifier (number) 3 is applied are shown as simple examples.

As to areas to which the identifiers are applied, no particular limitations are imposed insofar as the identifiers can be identified from images captured by a CCD camera or the like. When the beads have a core structure, the identifiers can be applied to the surfaces of their core structure portions (in this case, their shapes can be the same). The identifiers can also be applied to the surfaces of the beads irrespective of whether or not the beads have a core structure.

### (2) Production process of the bead set

A description will firstly be made about illustrative production of the beads which make up the above-described bead set 1 (see FIG. 1). It is possible to adopt a process that a light-curing resin is three-dimensionally pattern-exposed by a predetermined photofabrication exposure system, the pattern-exposed resin is immersed in a predetermined organic solvent to wash off the resin at uncured portions, and only the portions exposed to light and cured are allowed to remain to develop the three-dimensional pattern and hence to produce a desired three-dimensional structure.

The three-dimensional pattern can be converted into topographic data that its three-dimensional CAD data have been sliced into thin cross-sections, and using a light source composed of LEDs or LDs of ultraviolet wavelengths corresponding to the slice data, a two-dimensional pattern corresponding to the slice data is then formed by turning on and off respective pixels of DMD (Digital Micro-mirror Device). Through an objective lens, UV light of the two-dimensional pattern is irradiated onto a substrate on which the light-curing resin has been coated, so that the light-curing resin is pattern-exposed. Subsequent to the completion of the exposure of a specific slice layer, the light-curing resin corresponding to the next layer is coated as a stacked layer, and is then exposed to UV light of a pattern corresponding to the next layer. By repeating these steps, it is possible to perform three-dimensional pattern exposure corresponding to a stereoscopic rapid prototype.

When the resulting pattern-exposed block of the light-curing resin is immersed, subsequent to the exposure process, in a bath filled with such an organic solvent as causing dissolution of the unexposed and uncured resin portions, the desired three-dimensional structure can be obtained. As the pixel size of the DMD is as small as 15 µm or so, use of an objective lens having a magnifying power of 10 or so and a sufficiently high numerical aperture makes it possible to perform a pattern exposure on the order of micrometers (µm), so that stereoscopic rapid prototyping can be realized with an accuracy on the order of micrometers. It is to be noted that LCOS (Liquid Crystal on Silicon) may be used instead of DMD. Describing an example of the adoption of LCOS by using SXRD (Silicon-Xtal Reflective Display),

SXRD is a sort of reflective liquid crystal device that the orientation of each liquid crystal formed on a silicon mirror surface is controlled by an impressed voltage to change its reflectance. As its pixel size is as small as 7 to 9 µm or so, a resolution twice as high as that available from DMD can be expected.

As the construction of each bead, a cube (regular hexahedron) or a triangular pyramid (regular tetrahedron), 50 µm on a side, can be adopted. Further, beads of the construction that an identifier (e.g., convexities in the form of a bar code) is formed on each face are arranged as many as 100 beads in a matrix pattern on a flat surface. Even if 100 beads of the same type are arranged, they can be fully fitted within 1-mm squares. A multiplicity of types of beads having various stereoscopic shapes other than cube and regular tetrahedron and carrying different barcodes on their surfaces can be produced by the above-mentioned photofabrication process.

Subsequent to the production of the beads by the above-described photofabrication process, a metal such as Ni (nickel) is coated with a thickness of several tens nanometers or so on the surfaces of the beads by an electroless plating process so that visible light can be strongly reflected. In the electroless plating process, the beads are placed, for example, in an aqueous solution in which nickel sulfamate, palladium chloride and the like are dissolved, and by using a palladium colloid, Ni is caused to deposit on the surfaces of the beads.

The Ni-plated beads are next dispersed, for example, in a polystyrene/acetone solution, and the dispersion is added dropwise by a syringe into hexane under vigorous stirring to coat the surfaces of the beads with polystyrene. The resulting precipitate is collected by filtration, and is then re-dispersed in a methanol/water solution by using ultrasonic waves. By centrifugal separation, the lower layer is collected.

The collected layer is washed with methanol and then dried. The thus-dried beads are spread thin in a petri dish, followed by ozonation to form carboxyl groups on the surfaces of the beads. A mixed solution of EDC (ethylene dichloride; 100 mg/mL) and NHS (N-hydroxysuccinimide; 100 mg/mL) is added to the beads.

The mixed solution and the beads are reacted at room temperature for 30 minutes under shaking. The reaction product is collected by filtration and washed with water, and is then reacted, for example, with a synthetic oligomer(which has amino terminals and has a specific DNA base sequence)/NaCl (1M) solution. The resulting reaction product is collected by filtration and then dried to afford target beads surface-modified with the intended detection probe.

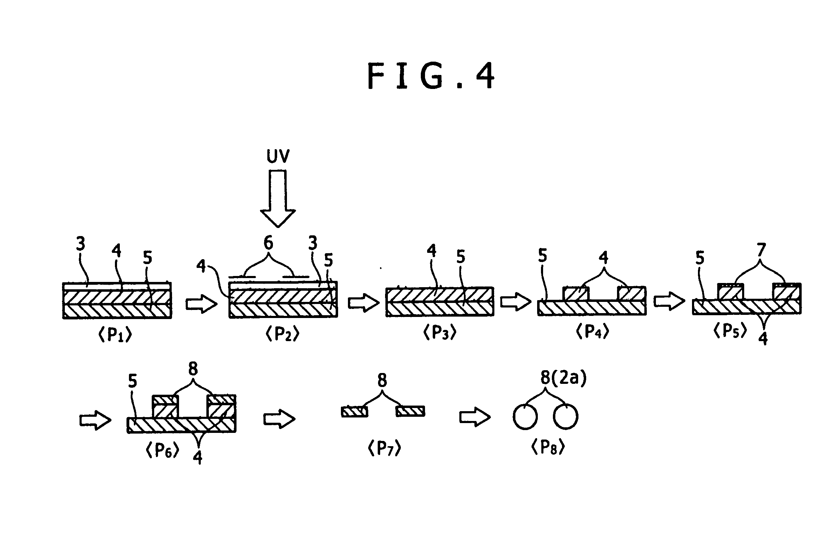

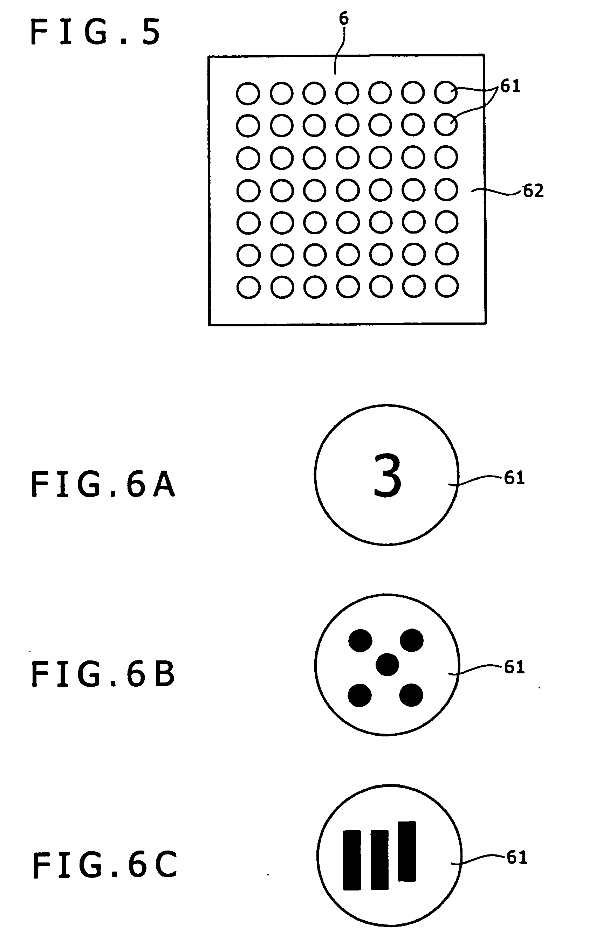

Referring to FIGS. 4 and 5, a description will next be made about one example of the process for the production of the beads that make up the bead set 2. The core portions 2a (see FIG. 2) of each different shape in the bead set 2 can be produced by applying electroless plating to a substrate on which a catalyst has been patterned, for example, by using a stamp patterned by photolithography.

The above production process will be described more specifically. FIG. 4 is a conceptual process-flow diagram of the process for the production of the bead set 1 according to the present invention. As shown in FIG. 4, a protective film 3 and a dry film resist 4 (e.g., "SUNFORT", product of Asahi Kasei Corporation) are firstly laminated on a substrate (e.g., glass-made substrate) 5 (see Step P₁ in FIG. 4). After a photomask 6 (e.g., a glass mask with circles, triangles, squares, pentagons, hexagons, starbursts or rectangles patterned thereon) is bonded on the protective film 3, UV exposure is performed (see Step P₂ in FIG. 4).

As an alternative, it is possible to contrive such that the core portions formed in the predetermined shape can be stripped from the substrate 5 by arranging a layer (a sacrificial layer formed of SiO₂ or MgO), which is removable with an acid or alkali, in place of the dry film resist 4 on the substrate 5.

FIG. 5 illustrates one example of a light-shielding pattern (or a light-transmitting pattern) on the photomask 6 employed upon forming circular core portions. In FIG. 5, respective circles 61 are light-transmitting regions, and a region 62 other than the circles indicates a light-shielding region. The size of each circle 61 can be designed, for example, to have a diameter of 50 µm.

By contriving to provide the inner part of each light-transmitting region (circle 61) on the photomask 6 with a light-shielding portion (non-exposing portion) formed of a single number, number string, character, character string, graphic, pattern (e.g., dot pattern), barcode or a combination of two or more of these identifiers, it is possible to produce core portions having the number, character or the like corresponding to the light-shielding portion.

FIGS. 6A to 6C are diagrams illustrating examples of each light-transmitting region (circle 61) arranged on the photomask 6 and provided with a light-shielding portion. FIG. 6A illustrates an example in which a light-shielding portion in the form of a single number is arranged, FIG. 6B shows another example in which a light-shielding portion in the form of a dot pattern is arranged, and FIG. 6C depicts a further example in which a light-shielding portion in the form of rectangular graphics (or a barcode) is arranged.

Referring back to FIG. 4, a description will now be made. After the above-described UV exposure step P₂, the protective film 3 and photomask 6 are stripped (see Step P₃ in FIG. 4), and subsequently, the unexposed portions are removed with an aqueous mild alkaline solution, followed by drying (see Step P₄ in FIG. 4). Next, an aqueous solution of palladium chloride (5 mg) and hydrochloric acid (0.1 mL) in purified water (20 mL) is prepared, is coated on convexities of the formed pattern, and is dried (see Step P₅ in FIG. 4).

As a method for forming a catalyst pattern 7, it is possible to fix, for example, a photosensitive catalyst for electroless plating (reference: "Technical Report 2000", p 52, Sumitomo Osaka Cement Co., Ltd.) or a palladium colloid on a substrate by light.

The substrate 5 with the catalyst pattern transferred on the convexities thereof is next immersed for 10 minutes in "BF-Ni Solution" (trade name, product of Khozai Corporation) which was controlled at 85°C (see Step P₆ in FIG. 4), washed with water, and then washed with strong alkali (see Step P₇ in FIG. 4). After the thus-stripped core portions 8 are collected by using a glass filter, they are dried together with the glass filter in a vacuum to obtain the core portions 8 for beads (see Step P₈ in FIG. 4). It is to be noted that the thus-obtained core portions 8 (which correspond to sign 2a in FIG. 2) are designed to have a thickness of about 2 µm, for example.

The core portions 8 obtained through Steps P₁ to P₈ as described above are added to a 1 wt% solution of polystyrene (molecular weight: 100,000) in acetone, and are thoroughly dispersed there. Into hexane under vigorous stirring, the dispersion is added dropwise from a syringe such that the core portions 8 are coated at surfaces thereof with polystyrene to form shell portions (which correspond to sign 2b in FIG. 2). The resulting precipitate is collected by filtration, and is then re-dispersed in a methanol/water solution by using ultrasonic waves. By centrifugal separation, the lower layer is collected. The collected layer is washed with methanol and is then dried.

### (3) Example making use of the beads

Taking, as an example, dry beads of the construction that they have as cores the core portions 8 obtained by the above-described production process, one application example (sequencing) of the dry beads will hereinafter be described based on an example.

The thus-obtained dry beads were spread thin in a petri dish, and by ozonation in an ozonation treatment system ("PDC200", trade name; manufactured by Yamato K.K.), carboxyl groups were formed on the surfaces of the beads. The beads were then added to a solution with EDC (ethylene dichloride) and NHS (N-hydroxysuccinimide) mixed at 100 mg/mL, respectively. Under shaking at room temperature, they were reacted for 30 minutes. The reaction product was collected by filtration and washed with water, and is then reacted with a DNA oligomer having amino terminals (as a primer)/NaCl (1M) solution. The resulting reaction product was collected and then dried to afford target beads.

As the core portions of beads, seven subsets were provided in total, for example, including a circle, triangle, square, pentagon, hexagon, starburst, and rectangle as graphics as viewed in front views. On the surfaces of the beads in the respective subsets, 7-mer DNA oligomers (as primers) of the base sequences shown below in Table 1 were immobilized through amide bonds, respectively. In this example, a 25-mer DNA oligomer the sequence of which was known (SEQ ID NO. 8: tccgataaca gtgatcagca tggct) was also provided beforehand as a target.

**Table 1**

| Shape of core portion | Base sequence of immobilized DNA oligomer | | | | | SEQ ID NO. |
|---|---|---|---|---|---|---|
| Circle | aggctat(1-7) | | | | | 1 |
| Triangle | | tattgtc(5-11) | | | | 2 |
| Square | | | gtcacta(9-15) | | | 3 |
| Pentagon | | | | tagtcgt(14-20) | | 4 |
| Hexagon | | | | | cgtaccg(18-24) | 5 |
| Starburst | aggctag(1-7, the 7^{th} base is a mismatch base) | | | | | 6 |
| Rectangle | ggcctta(mismatch sequence) | | | | | 7 |

After the above-described 7 subsets of beads were measured as much as 1 mg each and mixed together, ddNTP labeled with "CY^{™}3" (fluorescent dye, product of Amersham Biosciences Limited), "IPROOF DNA POLYMERASE" (trade name, product of Bio-Rad Laboratories, Inc.) and the target DNA oligomer were added, and "IPROOF HF BUFFER" (trade name, product of Bio-Rad Laboratories, Inc.) was added to adjust the concentrations of the ddNTP and target DNA oligomer at 200 mM and 1 µM, respectively.

After the resultant mixed solution was heated at 98°C for 10 seconds, the mixed solution was cooled down to 30°C and held at the same temperature for 1 minute. After the mixed solution was again held at 98°C for 10 seconds, centrifugation was conducted. A reaction was conducted in an Eppendorf tube, and an operation consisting of washing with 1 M NaCl and subsequent centrifugation was repeated three times. 1 M NaCl (10 µL) was added to the thus-obtained mixture, and the individual beads were measured for shape and fluorescence intensity.

When a method is adopted to attempt copying work of base sequences through the formation of a complementary chain (a polymerase extension reaction using primers) between the DNA oligomers (as primers) immobilized on the respective subsets of beads and the target DNA oligomer (SEQ ID No. 8) as a template, it may be contrived such that instead of the method making use of the ddNTP labeled with the fluorescent dye, a dye-quencher pair such as a fluorescent dye (e.g., fluorescein) and a quencher (e.g., "BHQ^{™}1", product of Biosearch Technologies, Inc.) are bonded beforehand to the 5'-positions and 3'-positions of the respective DNA oligomers immobilized on the beads, respectively, and that, as a result of the extension reactions using the immobilized DNA oligomers (as primers), the quencher is released and the quenching by the quencher is eliminated to emit fluorescence.

In essence, an intended analysis (e.g., the determination of the base sequence of a template DNA) can be performed by using such a method that the kind of a DNA oligomer (as a primer) to be immobilized is changed beforehand depending on the bead subset and a confirmation is performed as to with which bead subset or subsets the formation of a complementary chain (polymerase extension reaction using the primer) proceeds.

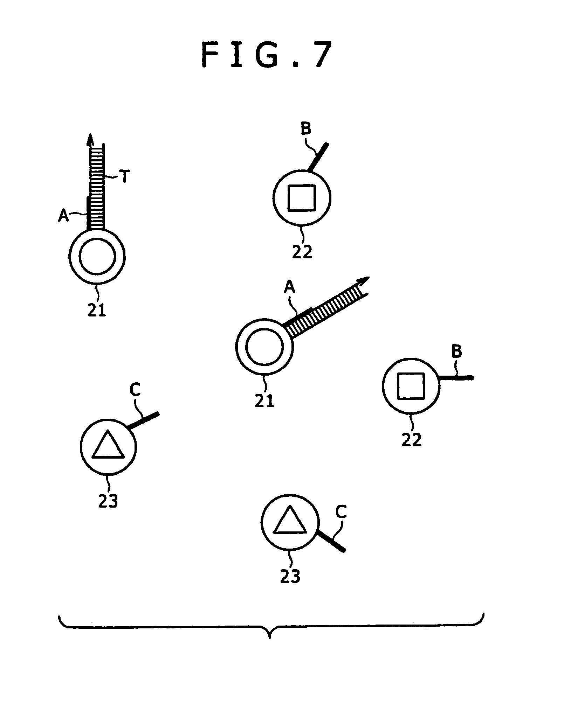

By way of example, FIG. 7 schematically illustrates a situation that DNA oligomers (as primers) A, B, and C of different base sequences have been immobilized on the three types of beads 21, 22, and 23, respectively, a target DNA (see sign T in FIG. 7) as a template for the DNA oligomer (as a primer) A immobilized on the beads 21, 21 having circular core portions among the beads 21, 22, 23 has been hybridized, and a polymerase extension reaction using the DNA oligomer (as a primer) A has then been allowed to proceed.

For example, by measuring the intensity of fluorescence originated from the ddNTP incorporated through the polymerase extension reaction and fluorescence from the fluorescent dye labeled on the DNA oligomer (as a primer) A and also specifying that these fluorescence is originated from the beads 21, it is possible to confirm that the target DNA as the template (see symbol T in FIG. 7) had at least a base sequence region capable of forming a complementary chain with the DNA oligomer (as a primer) A. When the quantity of a target DNA is small, it can be contrived to repeat a polymerase extension reaction using a primer.

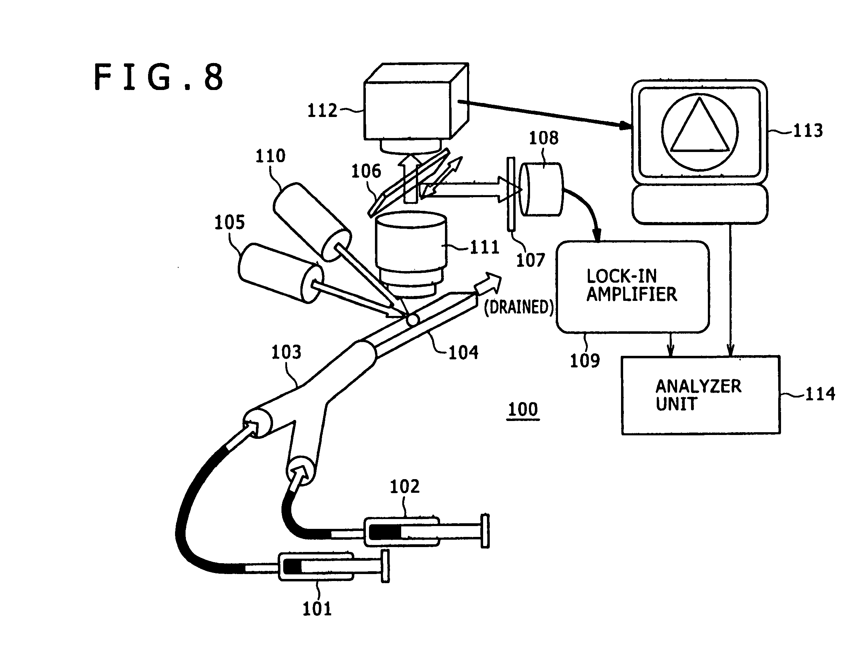

A description will hereinafter be made of an illustrative method for the measurement of bead shapes and an illustrative method for the measurement of fluorescence intensities. The measurements of bead shapes and fluorescence intensities can be performed, for example, by using a system 100 of the construction shown in FIG. 8.

This system 100 is constructed such that a syringe pump 101 for introducing a bead sample and another syringe pump 102 for feeding an aid solution to control a flow rate and the separation of beads are connected together by a Y-tube 103 and the bead sample is introduced into a flow cell 104 via the Y-tube 103. As the flow cell 104, an optical fiber connecting capillary tube (product of Nippon Electric Glass Co., Ltd.; inner diameter: 0.25 x 0.127 mm) can be used, for example.

A fluorescence excitation laser 105 arranged in a vicinity of the flow cell 104 is turned on to continuously monitor emission from the flow cell 104. This turn-on of the laser 105 is periodically effected, fluorescence is detected by PMT (photomultiplier tube) 108 via a dichroic mirror 106 and filter 107 arranged in a light traveling path, and subsequent to filtering processing and amplification, and the resulting fluorescence is imputed in a lock-in amplifier 109 to measure its intensity.

At a time point that the intensity of fluorescence begins to increase, an image detection laser 110 is turned on to capture the shapes of beads by a CCD camera 112 via a microscope 111, the beads are identified by a computer 113 based on their shapes from their captured images, and the image information is recorded in a storage unit of the computer 113. At an analyzer unit 114, the fluorescence intensity data and the image information are accumulated to analyze their correlation.

For increasing the accuracy of the image recognition, it may be contrived to perform the image-importing work a plurality of times until the intensity of fluorescence drops. By comparing the fluorescence intensity in each importing work with the corresponding value in the preceding importing work, taking the thus-determined maximum value as the fluorescence intensity of the bead and analyzing the image imported in the importing work corresponding to the maximum value, the type of the bead can be determined. The type of the bead and the maximum fluorescence intensity are then stored as a combination of values.

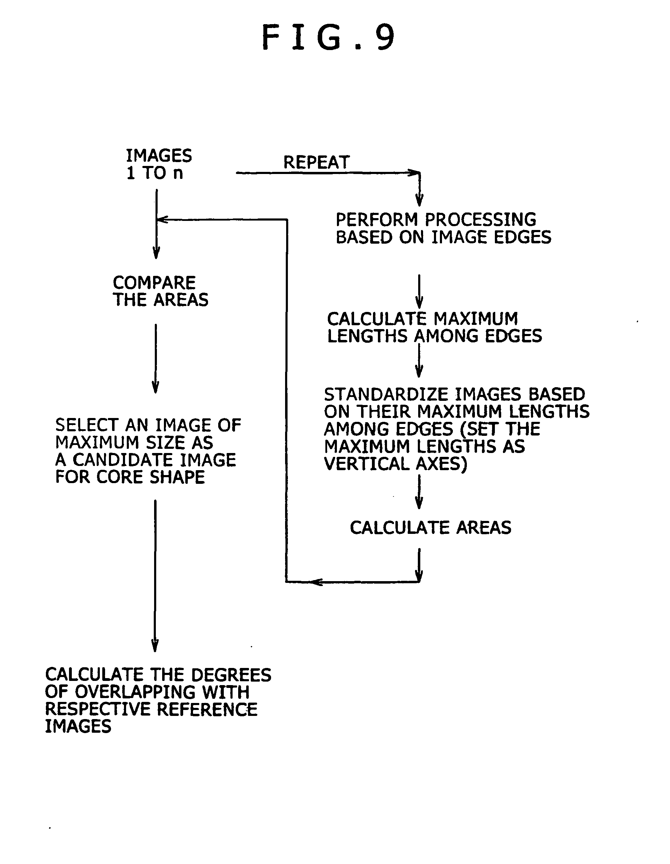

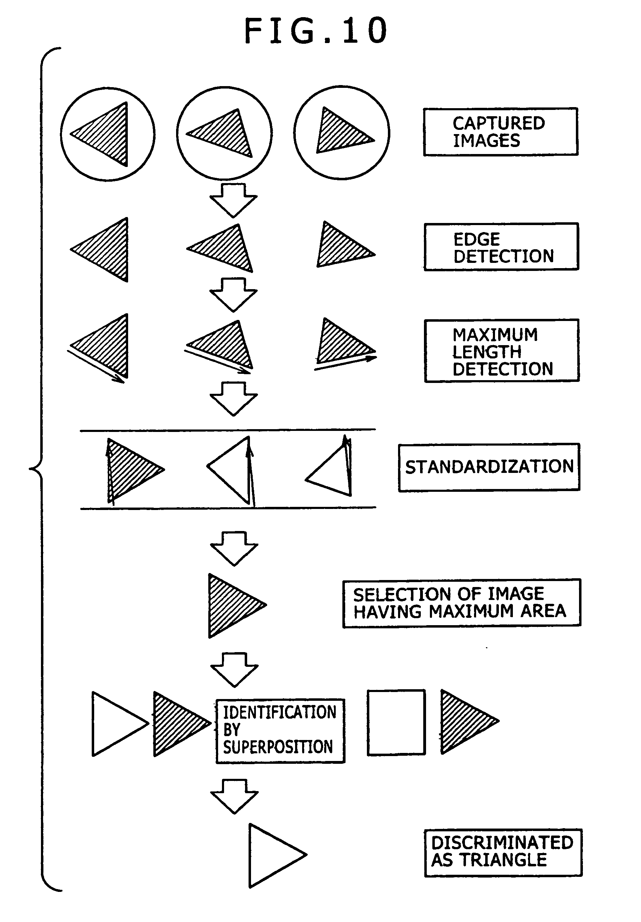

One example of a method for the identification of a bead shape will next be specifically described. The identification of a bead shape and its assessment can be performed by conducting superposition with a graphic as a reference. FIG. 9 is an illustrative flowchart relating to identification of a bead shape and its assessment, and FIG. 10 is a conceptual diagram of an illustrative procedure relating to the identification of the bead shape and its assessment.

Adopted in the illustrative identification method to be described hereinafter is a method that extracts the shape of the core portion of a bead by detecting edges of plural images acquired. It is to be noted that for the shape recognition of a bead, other methods such as the identification method relying upon the extraction of characteristic points may be used.

Edges of captured images are detected, the images of core portions are extracted, and then, the longest sides in the images are determined. Setting the longest sides as vertical axes, the image sizes are standardized. The captured images are compared with each other, and the image having the largest area is selected as a candidate image. A superposition of the candidate image with each reference image is performed, and the reference image that has given the largest overlap is discriminated (see FIG. 10 in particular). The maximum values of fluorescence intensity measured on the respective images are added to calculate the total quantity of fluorescence from the respective images.

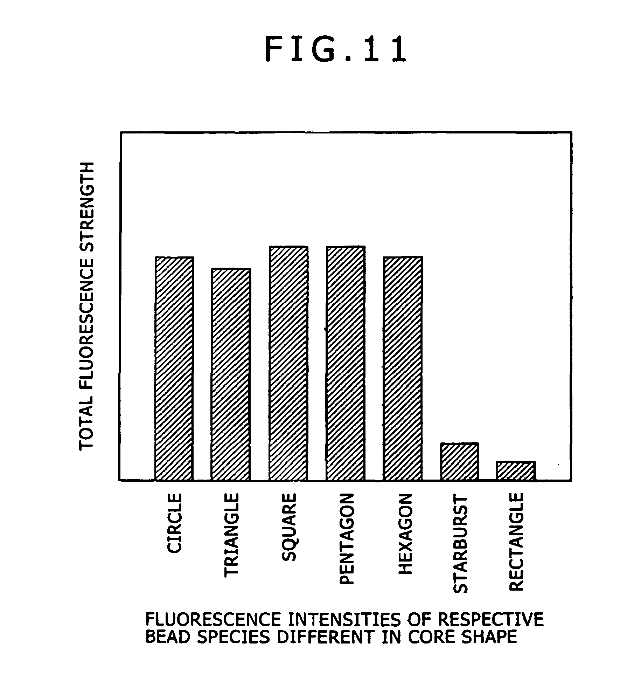

A description will next be made about illustrative work for the ascription of a base sequence. In FIG. 11, fluorescence intensities actually measured corresponding to totally seven types of beads, the types being different in the shape of each core portion, respectively, are histogrammatically presented. From the beads on which DNA oligomers, which were partial sequences of the template DNA (oligomer), were immobilized (those having circular, triangular, square, pentagonal and hexagonal shapes as the shapes of core portions as viewed in front views), substantially the same quantity of fluorescence was measured (see FIG. 11).

From the type of beads different in terminal base (those having a starburst shape in the shape of each core portion in front view) and the type of beads incapable of forming any complementary chain with the target DNA oligomer (template) (those having a rectangular shape in the shape of each core portion in front view), on the other hand, only a small quantity of fluorescence was observed (see FIG. 11).

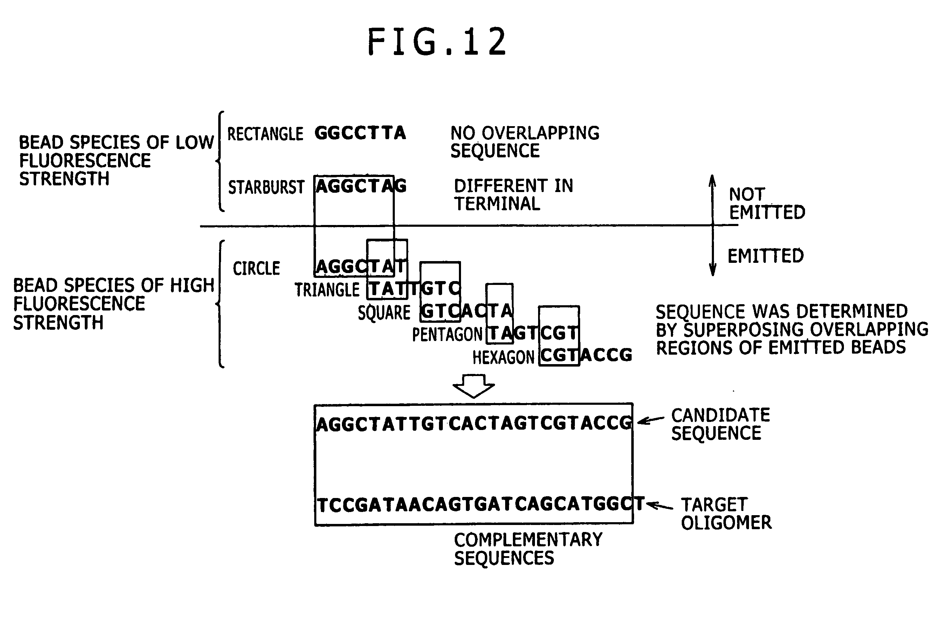

FIG. 12 is a schematic diagram illustrating a concept for determining the sequence of the target DNA oligomer by using the base sequences immobilized on the beads.

The sequence of the target DNA oligomer can be precisely determined by putting side by side the base sequences of the beads high in fluorescence intensity (see FIG. 12), superimposing the overlapping sequence regions to determine the sequence, and obtaining its complementary sequence.

In this example relating the determination of the sequence, the results from the use of the 7 kinds of base sequences (the results from the use of the 7 types of beads) have been specifically described. When 7 types of base sequences each consisting, for example, of 7 bases are used, there are combinations as many as 4 to the 7th power (16,384). By using beads of all the combinations, any given base sequence can be determined. When a base sequence is already known to a certain extent, the types of beads to be used can be made fewer as did in this example.

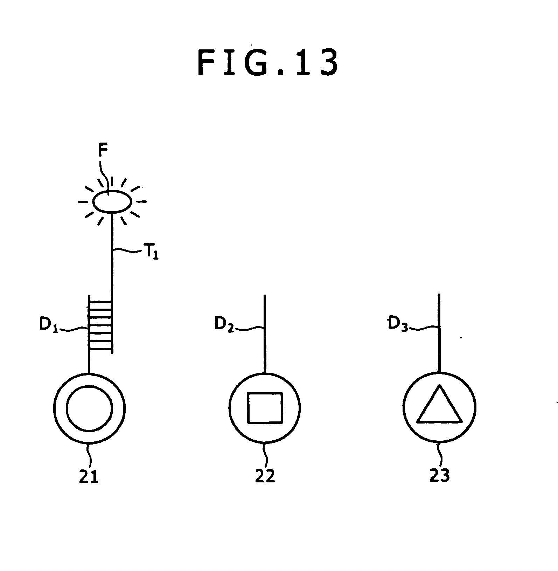

As shown in FIG. 13, for example, plural types of beads 21, 22, and 23 are added to a predetermined place of interaction. On the respective beads 21, 22, and 23, DNA probes D₁, D₂, and D₃ of different base sequences have been immobilized beforehand.

A target (e.g., cDNA) T₁ labeled with a fluorescent dye F is added to the place of interaction, and under predetermined appropriate conditions, hybridization is allowed to proceed. The results obtained by shape identification of the beads and the results obtained by a fluorescence intensity measurement are analyzed together. When fluorescence is found to be emitted, for example, only from the bead 21 as a result, the target T₁ is determined to have a complementary sequence to the DNA probe D₁ immobilized on the bead 21. In this manner, it is possible to find out, for example, the state of expression of a disease-related gene

When many kinds of DNAs (or RNAs) exist together in a sample, it possible to find the existence percentage of DNAs (or RNAs) having a specific partial sequence even if the analysis is not feasible up to the determination of their base sequences.

When a method of hybridization detection is used , no particular limitation is imposed on the detection method. Depending on the purpose, it is possible to freely choose, for example, the use of an intercalator that specifically binds to a double strand to emit fluorescence or the adoption of a detection method other than fluorescence detection (for example, detection relying upon a radioactive material, or electric detection) without being limited to the method that fluorescently labels a target.

## Claims

1. A method for determining a base sequence of a target nucleic acid strand, which comprises using at least a bead set composed of plural subsets of beads, wherein said beads in each subset are each provided with a surface permitting immobilization thereon of a substance that takes part in a predetermined corresponding reaction or interaction, and said individual beads in said bead set comprise core portions and shell portions as outer layers for said core portions, respectively, and said core portions are provided with shapes different from one subset to another, respectively, such that said individual beads in said bead set can be distinguished into said plural subsets through an analysis of said shapes of said core portions from information on captured images of said individual beads in said bead set by a computer, wherein DNA oligonucleotides as primers for a polymerase extension reaction are immobilized on the surfaces of the beads, wherein specific oligonucleotides are associated with a specific subset of the beads, a polymerase extension reaction is carried out either by using a ddNTP labelled with a fluorescent dye or by using a dye quencher pair bonded to the 5'-positions and 3'-positions of the respective DNA oligonucleotides immobilized on the beads, respectively, wherein as a result of the extension reaction using the immobilized DNA oligomers as primers, the quencher is eliminated to emit fluorescence, and wherein after detecting the intensity of fluorescence, the
shape of the beads is captured and the type of the bead and the maximum fluorescence intensity are then stored as a combination of values.

## Patentansprüche

1. Verfahren zur Bestimmung einer Basensequenz eines Ziel-Nukleinsäurestrangs, umfassend das Verwenden von mindestens einem Satz Kügelchen, der aus mehreren Teilsätzen von Kügelchen besteht, wobei sich auf den Kügelchen in jedem Teilsatz jeweils eine Oberfläche befindet, auf der eine Substanz immobilisiert werden kann, die an einer festgelegten entsprechenden Reaktion oder Wechselwirkung teilnimmt, und wobei die einzelnen Kügelchen in dem Satz Kügelchen jeweils Kernteile und Hüllenteile als Außenschichten für die Kernteile umfassen, und wobei die Kernteile Formen aufweisen, die sich jeweils von einem zum anderen Teilsatz unterscheiden, so dass die einzelnen Kügelchen in dem Satz Kügelchen in die mehreren Teilsätze unterschieden werden können, und zwar durch eine Analyse der Formen der Kernteile aus Information von aufgenommenen Bildern der einzelnen Kügelchen in dem Satz Kügelchen durch einen Computer, wobei DNA-Oligonukleotide als Primer für eine Polymerase-Verlängerungsreaktion auf den Oberflächen der Kügelchen immobilisiert werden, wobei spezifische Oligonukleotide mit einem spezifischen Teilsatz von Kügelchen assoziiert sind, eine Polymerase-Verlängerungsreaktion entweder durch Verwendung eines ddNTP, das mit einem Fluoreszenzfarbstoff markiert ist, oder durch Verwendung eines Farbstoffquencherpaars erfolgt, das an die 5'-Positionen und 3'-Positionen der jeweiligen DNA-Oligonukleotide, die jeweils auf den Kügelchen immobilisiert sind, gebunden ist, wobei als Folge der Verlängerungsreaktion mit den immobilisierten DNA-Oligomeren als Primer der Quencher eliminiert wird und Fluoreszenz aussendet, und wobei nach dem Nachweisen der Fluoreszenzintensität die Form der Kügelchen aufgenommen wird und dann der Typ des Kügelchens und die maximale Fluoreszenzintensität als Kombination von Werten gespeichert wird.

## Revendications

1. Procédé pour déterminer une séquence de base d'un brin d'acide nucléique cible, qui comprend l'utilisation d'au moins un ensemble de billes composé d'une pluralité de sous-ensembles de billes, lesdites billes dans chaque sous-ensemble étant chacune pourvue d'une surface permettant l'immobilisation sur celle-ci d'une substance qui participe à une réaction d'interaction correspondante prédéterminée, et lesdites billes individuelles dans ledit ensemble de billes comprenant des parties de noyau et des parties d'enveloppe en tant que couches externes pour lesdites parties de noyau, respectivement, et lesdites parties de noyau étant pourvues de formes différentes d'un sous-ensemble à un autre, respectivement, de sorte que lesdites billes individuelles dans ledit ensemble de billes puissent être distinguées dans ladite pluralité de sous-ensembles par une analyse desdites formes desdites parties de noyau à partir d'informations sur des images capturées desdites billes individuelles dans ledit ensemble de billes par un ordinateur, des oligonucléotides d'ADN en tant qu'amorces pour une réaction d'extension par polymérase étant immobilisés sur les surfaces des billes, des oligonucléotides spécifiques étant associés à un sous-ensemble spécifique des billes, une réaction d'extension par polymérase étant conduite en utilisant un ddNTP marqué avec un colorant fluorescent ou en utilisant une paire colorant-inactivateur liée aux positions 5' et aux positions 3' des oligonucléotides d'ADN respectif immobilisés sur les billes, respectivement, où, en conséquence de la réaction d'extension utilisant les oligomères d'ADN immobilisés en tant qu'amorces, l'inactivateur est éliminé pour émettre une fluorescence et où, après la détection de l'intensité de fluorescence, la forme des billes est capturée et le type de la bille et l'intensité maximale de fluorescence sont ensuite stockés sous forme de combinaison de valeurs.
